# EUROPEAN PATENT SPECIFICATION

(11) **EP 2 883 553 B1**
(45) Date of publication and mention of the grant of the patent: **12.12.2018**
(21) Application number: 13879455.7
(22) Date of filing: 07.08.2013
(51) Int. Cl.: A61L 27/06, A61L 27/50, A61C 8/00, B08B 3/08, C23G 1/10, C23G 3/00, A61L 2/18

(54) **PROCESSING METHOD AND PROCESSING DEVICE FOR IMPLANT MATERIAL HAVING EXCELLENT BIOCOMPATIBILITY**
VERARBEITUNGSVERFAHREN UND VERARBEITUNGSVORRICHTUNG FÜR EIN IMPLANTATMATERIAL MIT HERVORRAGENDER BIOKOMPATIBILITÄT
PROCÉDÉ DE TRAITEMENT ET DISPOSITIF DE TRAITEMENT POUR MATÉRIAU D'IMPLANT PRÉSENTANT UNE EXCELLENTE BIOCOMPATIBILITÉ

(30) Priority: 13.08.2012 JP 2012179257
(43) Date of publication of application: 17.06.2015
(73) Proprietor: Aichi Gakuin, Aichi 464-8650 (JP); De Nora Permelec Ltd, Fujisawa-shi, Kanagawa 252-0816 (JP)
(72) Inventor: MURAKAMI, Hiroshi, Nagoya-shi Aichi 464-8650 (JP); ICHIKAWA, Kazuhiro, Fujisawa-shi Kanagawa 252-0816 (JP); NISHIKI, Yoshinori, Fujisawa-shi Kanagawa 252-0816 (JP)
(74) Representative: Blodig, Wolfgang
(86) International application number: PCT/JP2013/071397
(87) International publication number: WO 2014/027595

(56) References cited:
- EP-A2- 0 988 834
- WO-A1-2013/081290
- JP-A- 2003 226 987
- JP-A- 2003 239 088
- JP-A- 2005 095 584
- JP-A- 2006 346 203
- JP-A- 2007 125 548
- JP-A- 2008 073 604
- JP-A- 2009 138 262
- JP-A- 2011 079 502
- JP-A- 2012 005 658
- JP-A- 2012 075 749
- JP-A- 2012 107 270
- US-A1- 2005 161 120
- HIROSHI ET AL: 'OZONE-SUI RIYO NO GUIDELINE SAKUTEI NI MUKETE: DENTAL IMPLANT NI OKERU ONZONE-SUI TEKIYOREI' BULLTIN OF JAPAN RESEARCH SOCIETY FOR MEDICAL AND HYGIENIC USE OF OZONE vol. 19, no. 2, 31 May 2012, pages 58 - 63, XP008176870
- HIROSHI ET AL: 'SHIKA RINSHO NI OKERU OZONE-SUI NO RIYA OZONE-SUI TO DENKAISUI TONO HIKAKU TO SONO KAIHATSU KEII' BULLTIN OF JAPAN RESEARCH SOCIETY FOR MEDICAL AND HYGIENIC USE OF OZONE vol. 19, no. 1, 19 February 2012, pages 18 - 26, XP008176871
- REI KAWAMURA ET AL: 'TITANIUM IMPLANT TAIHYOMEN NO TANSO KAGOBITSU NI TAISURU OZONE-SUI SENJO NO KOKA' JAPAN RESEARCH SOCIETY FOR MEDICAL AND HYGIENIC USE OF OZONE 21 April 2013, pages 13 - 15, XP008176872
- HIROSHI ET AL: 'TITANIUM HYMOMEN NO TANSO KAGOBUTSU NI TAISURU OZONE-SUI SENJO NO KOKA' JOURNAL OF JAPANESE SOCIETY OF ORAL IMPLANTOLOGY vol. 25, 10 September 2012, pages 171 - 181, XP008176869

## Description

### Technical Field

The present invention relates to a method for treating an implant material having excellent biocompatibility, and particularly to a method for treating a dental implant material.

### Background Art

It is important in dental implant surgery that an implant is sufficiently bound with a bone (osseointegration). As an implant material to be used for inserting into the back of body, titanium or alloys containing titanium as a base have been used, and the characteristic is that the materials have excellent biocompatibility and the time required for the materials to be integrated with the bone can be shortened. However, since sufficient binding is not formed in the back of body with a titanium implant having a smooth surface structure, it is necessary to roughen the surface. In Patent Literatures 1 and 2, a method in which a macro-roughened surface is formed on a surface of the implant material made of titanium or an alloy containing titanium as a base by sand blast in the first process, and subsequently a micro-roughened surface is formed on the surface of the macro-roughened surface by means of treatment in an acid bath has been proposed. It is described that the pore size of the macro-roughened surface is set to be larger than 10 µm, preferably 20 µm or larger and that the pore size of the micro-roughened surface is set to from 0.5 µm to 2 µm.

Moreover, as described in Non Patent Literature 1, a surface of a titanium metal is naturally oxidized in air and water, and it is inferred that a layer made from a hydroxy group and oxygen is formed in the outermost atomic layer of the titanium surface through reaction with water. However, the titanium surface is contaminated by easily volatile hydrocarbons and other compounds which are present in air and is liable to become hydrophobic, and with such a contaminated implant material, it is not possible to form a strong bonding by coalescing with a bone material in a short time. Moreover, as the time during which the implant material is exposed to air is longer, the contaminated layer becomes thicker. In the same way, it is essential to keep the surface of the implant material clean and maintain the hydrophilicity on the surface of the implant material even at the time of implanting the implant material.

The implant material to which hydrophilicity is imparted and sterilization treatment is applied is stored in a sealed container filled with a gas that does not give an impact to biological activity, for example, nitrogen, oxygen, and a rare gas such as argon. It is preferable that the sealed container is not permeable to a gas or liquid. It is preferable that the airtight and watertight sealed container is produced from glass, a metal, a synthesized polymer, or another airtight and watertight material, or that the airtight and watertight sealed container is a sealed ampoule made of a combination of these materials.

Even when the sealed container is open just before the implantation of the implant material, it is difficult to prevent deterioration in hydrophilicity of the surface of the implant material because the surface of the implant material makes contact with the atmosphere outside anyway. Accordingly, development of a production or treatment method by which the hydrophilicity on a hydrophilic implant surface that is subjected to hydro-oxidation is substantially maintained over a long period of time even when the contact with air occurs has been expected.

An artificial root of tooth (dental implant) that is used for implant treatment is made of titanium or a titanium alloy as described above, and according to a recent study (the study made by a group of Takahiro Ogawa, Associate Professor at UCLA, USA), carbon in air is adhered to the surface of titanium from immediately after the titanium is produced and has a serious impact on the adhesion strength to cells. It is understood that the ability of titanium to bind with bones is reduced to a half when one month is passed after production and the ability continues to decrease. The present inventors consider that the phenomenon needs to be improved because there is a risk that the following problems are brought about.
1) There is a possibility that biological aging is under way or has been completed at the time of use of an implant material, and therefore it is apprehended that the implant material is not used in the best condition.
2) It is extremely difficult to know the date of production of an implant material when the implant material is implanted, and therefore it is unknown to what extent does the titanium surface is deteriorated.
3) Thus, there is a possibility that differences in ability to bind with bones arise depending on the products even though the products are of the same brand.

In order to solve these problems, a method for recovering the binding ability of titanium by irradiating the implant with an ultraviolet ray (UV) having a specific wavelength to remove the adhered carbon. It has been reported that the ability of the implant to bind with bones is enhanced 3 times at an early stage of implantation, and finally 1.8 times by the method when compared with the ability to bind with bones in the case where irradiation with UV is not conducted.

In Patent Literature 3, a method for obtaining a hydrophilic surface subjected to hydro-oxidation by treating with a high energy ultraviolet ray to a surface of an implant material is disclosed. The advantage of the method is that further sterilization of the implant is unnecessary. The high energy UV ray that is used in the invention of Patent Literature 3 involves: UV light having a wavelength within a range from 150 nm to 300 nm, preferably within a range from 170 nm and 260 nm; particularly UV light having a wavelength within a range of absorption maximum of oxygen, in other words, a wavelength of about 184.9 nm; and UV light having a wavelength within a range of absorption maximum of ozone, in other words a wavelength of about 253.7 nm, and with UV light having the wavelength in the above-described ranges, formation and decomposition of ozone are simultaneously facilitated, and high energy chemical bond such as, for example, ethylenic carbon-carbon double bond can be destroyed.

Since the strength of the above-described radiation ray decreases exponentially depending on the distance from a UV generation source, it is advantageous to keep a distance between the implant surface to be treated and the UV generation source short, for example within a range of about 1 mm. The time to be required for UV treatment depends on the nature and thickness of the contaminated layer, and it is preferable that the radiation time is from 1 minute to 15 minutes.

As an apparatus that removes organic compounds on the surface of the artificial root of tooth, the following publicly known technologies are present other than the above-described technology. For example, Patent Literature 4 relates to a cleaning treatment apparatus for a bio-implant, the apparatus applying cleaning treatment to the bio-implant by irradiating the bio-implant with a UV ray and simultaneously allowing the bio-implant to contact with ozone.

Moreover, Patent Literature 5 relates to a light irradiation apparatus for a dental implant, the apparatus irradiating with a UV ray a screw type dental implant having a screw part made of spiral protrusion.

The light irradiation apparatus irradiating the dental implant with an ultraviolet ray can surely irradiate the screw part to be implanted to an alveolar bone or the like with an ultraviolet ray, and since a plurality of dental implants are usually used in one dental implant surgery, a light irradiation apparatus suitable for irradiating a plurality of screw type dental implants with an ultraviolet ray is developed.

Furthermore, in Patent Literatures 6 and 7, the structure of an organic contaminant removal apparatus for an artificial root of tooth, the organic contaminant removal apparatus using an excimer lamp radiating UV light, in which apparatus: UV light from the excimer lamp is not irradiated to an operator; it is unnecessary to worry about an influence by generated ozone; and UV light cleaning of an artificial root of tooth can be conducted effectively and safely is described.

However, in the methods for treating the implant material with a UV ray as described in the above-described Patent Literatures 4 to 7, complete sterilization and treatment for decomposing carbides cannot sufficiently be conducted particularly at recessed parts on the surface for an implant component having complicated unevenness parts. This is because existence of active species generated by ultraviolet light, such as ozone is limited only at parts where irradiation is conducted, and there is a risk that the treatment becomes incomplete depending on the shape of the surface.

Moreover, the treatment apparatuses for an implant material by an ultraviolet ray as described in the above-described Patent Literatures 4 to 7 are not practical because the apparatuses become expensive, and it is described that the hydrophilicity is maintained for about 1 hour after irradiation, however since the titanium surface is easily contaminated in the implant surgery process, the hydrophilicity for about 1 hour cannot be assured.

Furthermore, since selection of an appropriate root of tooth in implant surgery of a person in therapy cannot be carried out in a clean storage state but is carried out in usual living space, the surface contamination is unavoidable. Moreover, in the above-described apparatuses, since ozone in the apparatus is discharged and a sample whose temperature is elevated by ultraviolet ray irradiation is cooled before taking out the implant, waiting time becomes necessary, and therefore continuous use has been difficult, which has been a problem.

In Patent Literature 8, a dental cleaning apparatus using ozone water is disclosed. The apparatus is a dental ozone water cleaning apparatus for supplying ozone water into oral cavity of a patient to conduct cleaning, the apparatus including: an ozone water production device producing ozone water; and a dental handpiece being connected to the ozone water production device through a tube and having dimensions capable of being inserted into oral cavity, in which apparatus ozone water that is supplied from the ozone water production device is made so as to be ejected from an ejection nozzle provided to the dental handpiece toward a desired site in the oral cavity. When disinfection and sterilization can be applied by allowing ozone to directly reach the desired site in the oral cavity in such a way as described above, the apparatus exhibits a high level of effectiveness in preventing a periodontal disease due to anaerobic bacteria and infectious disease infected from the site of surgery to which the prevention effectiveness has been insufficient in the past. Furthermore, there is a possibility that various microorganisms are contained in waste liquid discharged from the oral cavity through a vacuum tube or the like, however when the ozone water cleaning is conducted, the microorganisms in waste liquid are deactivated by a large amount of ozonized water that is ejected into the oral cavity, and therefore it becomes possible to dispose of the waste liquid as it is.

However, with only the dental cleaning apparatus using ozone water according to Patent Literature 8, the contact time of ozone water with the implant material is limited to only during the surgery, and cleanliness of the surface of the implant material can be maintained, however the contact time is short and is insufficient in terms of time, and therefore a large amount of carbides adhered to the surface of the implant material that is stored in the atmosphere for a long period of time cannot be reduced, and the biocompatibility on the surface of the titanium implant having a complicated surface form cannot be maintained or recovered.

Moreover, Patent Literature 9 shows an apparatus for producing ozone water by an electrolysis system, it is disclosed that conductive diamond is used as an electrode, and, in particular, an electrolysis cell obtained by disposing a band-shaped diaphragm member on a bar-shaped conductive diamond electrode and further disposing a wire-shaped counter electrode thereon is disclosed. Moreover, a membrane-electrode assembly disclosed in Patent Literatures 10 and 11 is characterized in that: a diaphragm such as an ion exchange membrane is provided around a bar-shaped or cylindrical electrode, usually anode (hereinafter, also referred to as bar-shaped anode); a wire-shaped counter electrode or a porous counter electrode, usually a wire-shaped cathode or a porous cathode, is disposed around the membrane; and the membrane and the anode are fixed using the cathode so that at least a part of the membrane and a part of the anode make contact with each other and at least a part of the membrane and a part of the cathode make contact with each other, thereby forming an anode chamber having a gas/liquid channel between the membrane and the anode, further preferably between a plurality of anodes.

In Patent Literatures 9, 10, and 11, an example of the apparatus for producing ozone water by an electrolysis system is shown, and electrolyzed ozone water that would be suitable for surface treatment of the implant material by the present invention can be produced, however it is not disclosed at all about whether or not the electrolyzed ozone water can be applied to the surface treatment of the implant material.

### Citation List

### Patent Literature

Patent Literature 1: Japanese Patent Laid-Open No. 3-47264 (Japanese Patent No. 3047373)
Patent Literature 2: EP 0388576
Patent Literature 3: National Publication of International Patent Application No. 2005-505352
Patent Literature 4: Japanese Patent Laid-Open No. 2012-75549
Patent Literature 5: Japanese Patent Laid-Open No. 2012-75548
Patent Literature 6: Japanese Patent Laid-Open No. 2012-119
Patent Literature 7: Japanese Patent Laid-Open No. 2012-120
Patent Literature 8: Japanese Patent Laid-Open No. 2012-5658
Patent Literature 9: Japanese Patent Laid-Open No. 2006-346203 (Japanese Patent No. 4410155)
Patent Literature 10: Japanese Patent Laid-Open No. 2009-138262 (Japanese Patent No. 4723627)
Patent Literature 11: Japanese Patent Laid-Open No. 2008-73604 (Japanese Patent No. 4980016)

### Non Patent Literature

Non Patent Literature 1: H. P. Boehm, Acidic and Basic Properties of Hydroxylated Metal Oxide Surfaces, Discussions Faraday Society, Vol. 52, 1971, p 264-275

EP 988834 discloses a device for use in disinfecting treatment. JP 2001 79502 A discloses an apparatus for cleaning using ozone water.

### Summary of Invention

### Technical Problem

In order to solve the problems, studies on a method for maintaining or recovering biocompatibility on a surface of a titanium implant having a complicated surface form have been made, and as a result thereof, in the present invention, an apparatus having a surface oxidation treatment mechanism with high-concentration ozone water and an ozone water-supplying mechanism during surgery has been devised and it has been found out that allowing the implant to contact with the ozone water in the apparatus for a certain period of time becomes an economically excellent treatment method that is capable of reducing carbide even for a sample stored in the atmosphere for a long period of time, recovering the biocompatibility, and imparting a bactericidal property to ozone water as cleaning water at the site of surgery. The apparatus is inexpensive as compared with conventional apparatuses and is excellent in practicability as an apparatus capable of applying continuous treatment.

### Solution to Problem

In order to achieve the above-described objects, the solution to the problems the present invention provides a method according to claim 1 to 6, in particular for treating an implant material having excellent biocompatibility, the method comprising:
immersing a surface-roughened implant material comprising titanium or a titanium alloy in electrolyzed ozone water; and
maintaining the electrolyzed ozone water at normal temperature,
thereby treating the implant material by the electrolyzed ozone water and preventing contamination due to adsorption of carbide on the surface of the implant material and imparting hydrophilicity to the surface of the implant material.

In a preferred embodiment the present invention provides a method for treating an implant material having excellent biocompatibility, the method further comprising:
supplying electrolyzed ozone water having an ozone concentration of from 0.2 ppm to 5 ppm to the surface of the implant material just before and during the implant surgery, thereby preventing contamination due to adsorption of carbide on the surface of the implant material and imparting hydrophilicity to the surface of the implant material.

In order to achieve the above-described objects, the carbon content in the carbide adsorbed on the surface of the implant material after treatment is made less than 25 atomic%.

Preferably, the carbon content in the carbide adsorbed on the surface of the implant material after treatment is made less than 20 atomic%.

In the method for treating an implant material having excellent biocompatibility ozone water having an ozone concentration of from 1 ppm to 25 ppm is used as the electrolyzed ozone water in which the implant material is immersed.

Preferably, ozone water having an ozone concentration of from 0.3 ppm to 5 ppm is used as the electrolyzed ozone water supplied to the surface of the implant material just before the implant material is implanted.

Preferably, the implant material is an implant material which has lost biocompatibility due to contact with air for a long period of time.

Preferably, the implant material is a dental implant material.

### Advantageous Effects of Invention

According to the present invention, a biocompatible implant material comprising titanium or a titanium alloy and having a roughened surface suitable for implantation, wherein contamination due to adsorption of carbide produced before or during surgery on the surface of the implant material is prevented and hydrophilicity is imparted can be obtained by allowing the implant material to contact with electrolyzed ozone water on a steady condition.

Moreover, according to the present invention, an implant material which has been stored while being contacted with air for a long period of time and has lost biocompatibility can be revitalized.

Furthermore, utilization of the favorable effect of ozone on the surface of the implant material has so far been expected, however an adequate ozone water production apparatus has not been available and therefore the utilization of ozone has not been put into practical use yet. In the present invention, when electrolyzed ozone water that is produced with an electrochemical ozone water production apparatus is utilized, high-concentration ozone water is produced easily and instantaneously by using a small-sized and excellent electrolytic ozone water apparatus having a catalyst and an electrolysis cell, and almost entire surface of the implant material can be oxidized and disinfected by immersing the implant material to be a target in the high-concentration ozone water on a steady condition under running water in a short time. Furthermore, the progress of osseointegration is further facilitated by conducting the implant surgery just after the immersion while ejecting ozone water. Although the ozone gas is generated to some extent, the treatment mainly using ozone water in an enclosed space is possible and therefore safety to operators can be secured.

### Brief Description of Drawings

[Figure 1] Figure 1 is a diagram illustrating an aspect of a flow sheet showing the entire constitution of implant surgery using a method for treating an implant material according to the present invention.
[Figure 2] Figure 2 is a flow sheet illustrating an example of an ozone water cleaning apparatus to be used for a method for treating an implant material according to the present invention.
[Figure 3] Figure 3 is a cross section view illustrating an aspect of an electrolytic ozone water production apparatus to be used for a method for treating an implant material according to the present invention.
[Figure 4] Figure 4 is a graph showing an implant material-treating effect with ozone water according to the present invention.

### Description of Embodiments

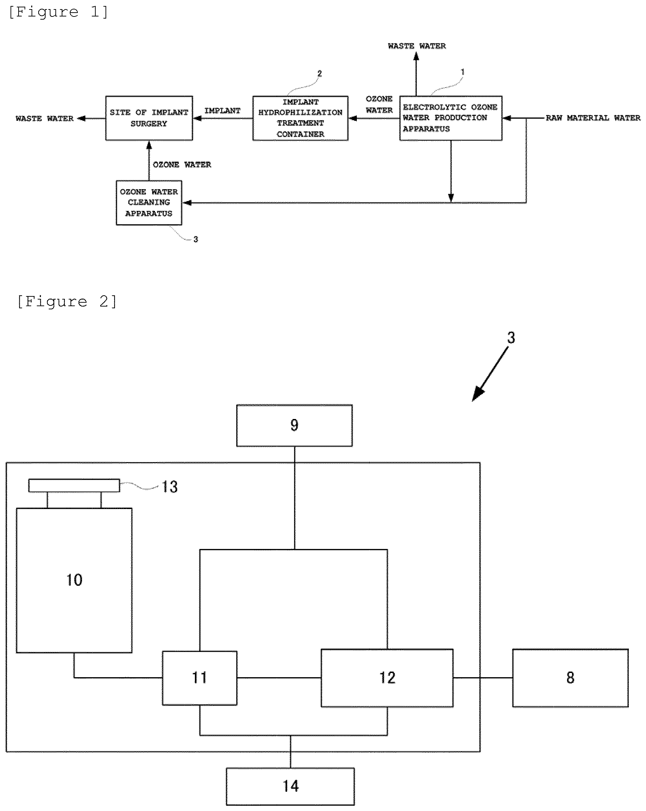

Hereinafter, the aspect of the present invention will be described based on Figure 1.

Figure 1 illustrates an aspect of a flow sheet showing the entire constitution of implant surgery using a method for treating an implant material according to the present invention, and reference numeral 1 denotes an electrolytic ozone water production apparatus producing electrolyzed ozone water by electrolysis, reference numeral 2 denotes an implant hydrophilization treatment container in which hydrophilization treatment is applied to an implant material by storing the electrolyzed ozone water that is produced with the electrolytic ozone water production apparatus 1, immersing a surface-roughened implant material comprising titanium or a titanium alloy until just before the implant surgery, and maintaining the implant material at normal temperature, and reference numeral 3 denotes an ozone water cleaning apparatus taking out the implant material from the implant hydrophilization treatment container 2 just before the implant surgery, and thereafter supplying, during the implant surgery, the electrolyzed ozone water that is produced with the electrolytic ozone water production apparatus 1 to the surface of the implant material that is set at the site of the implant surgery.

In the present invention, first of all, tap water or purified water is fed to the electrolytic ozone water production apparatus 1, and the electrolyzed ozone water is produced with the electrolytic ozone water production apparatus 1. The electrolyzed ozone water that is produced is fed to the implant hydrophilization treatment container 2 and stored in the implant hydrophilization treatment container 2, and the surface-roughened implant material comprising titanium or a titanium alloy is immersed in the electrolyzed ozone water until just before the implant surgery and is held at normal temperature, thereby preventing contamination due to adsorption of carbide on the surface of the implant material and imparting hydrophilicity to the implant material.

As the electrolyzed ozone water in which the implant material is to be immersed, ozone water having an ozone concentration of from 1 ppm to 25 ppm is used.

As electrolysis conditions, it is preferable from the standpoint of stability and activity of produced substances that the temperature is from 5°C to 40°C, and it is preferable that the direct current for applying to an electrolysis cell is 0.01 to 1 A/cm² in terms of current density to a plane of projection for an anode. The flow rate of water in this case is adjusted so that an intended ozone concentration is obtained. It is possible to complete the hydrophilization treatment in a shorter time as the concentration of ozone water is higher, and the concentration is from 1 ppm to 25 ppm. For example, the flow rate of water may be changed from 1 L/min to 50 mL/min in order to adjust the ozone concentration from 1 ppm to 25 ppm for a cell to which a current of 1 A can be applied.

Removal of carbide with ozone water can be completed in a short time when the concentration of ozone water is high, and a treatment time of 5 to 10 minutes is most preferable. Moreover, it is preferable to maintain the temperature of the implant material at around room temperature (about 25°C) when used in oral cavity as it is because a burden to patients is too large when the implant material is too cold. The carbon content in the carbide adsorbed on the surface of the implant material is made less than 25 atomic%, preferably less than 20 atomic%. Here, the carbon content (atomic%) in the carbide adsorbed on the surface of the implant material is determined by conducting surface analysis to measure the concentration of elements in a volume of φ 100 µm × 3 nm on the sample surface by XPS (product name "XPS-PHI5000 Versa Probe" manufactured by ULVAC-PHI, INCORPORATED).

The contact time of the implant material with ozone water that is necessary for hydrophilization of the surface of the implant material in the implant hydrophilization treatment container 2 takes from 1 minute to 60 minutes, although the contact time depends on the contamination level of the implant material. It is preferable to use ultrasonic treatment together with the hydrophilization treatment by ozone water in order to shorten the treatment time.

Furthermore, the contamination due to adsorption of carbide on the surface of the implant material is prevented and the hydrophilicity is imparted to the implant material even during the implant surgery by taking out the implant material from the implant hydrophilization treatment container 2 just before the implant surgery and thereafter supplying electrolyzed ozone water that is supplied from the ozone water cleaning apparatus 3 during the implant surgery to the surface of the implant material that is set at the site of the implant surgery.

Ozone water that has been used for implant surgery is discharged from a vacuum tube provided in the mouth. In this case, the ozone gas concentration in oral cavity is diluted to a level of 0.1 ppm or less, which is below the labor standard, and it is possible to avoid a case that a patient inhales the ozone gas by mistake. In order for this to achieve, it is preferable to make the concentration of ozone water from 0.2 ppm to 5 ppm.

As described above, it is preferable from the standpoint of preventing the contamination due to adsorption of carbide on the surface of the implant material that the concentration of the electrolyzed ozone water in which the implant material is immersed until just before the implant surgery and which is produced with the electrolytic ozone water production apparatus 1 is high, and, on the other hand, it is not preferable that the concentration of the ozone water supplied to the surface of the implant material that is set at the site of the implant surgery during the implant surgery after taking out the implant material from the implant hydrophilization treatment container 2 just before the implant surgery is made high from the standpoint of the Industrial Safety and Health Act.

In addition, the electrolytic ozone water production apparatus 1 and the ozone water cleaning apparatus 3 are described as individual apparatuses because the concentrations of ozone water produced with the respective apparatuses are different, however the electrolytic ozone water production apparatus 1 can also be used as an electrolytic ozone water production apparatus for the ozone water cleaning apparatus 3. In this case, the amount of ozone production may be adjusted in accordance with the purpose by providing a control apparatus.

Figure 2 illustrates a flow sheet showing an example of the ozone water cleaning apparatus 3.

In the present invention, the implant material is set at the site of the implant surgery when the implant surgery is conducted after taking out the implant material from the implant hydrophilization treatment container 2 just before the implant surgery, and the electrolyzed ozone water that is produced with the ozone water cleaning apparatus 3 is supplied to the surface of the implant material. It is preferable that the ozone water cleaning apparatus 3 is connected to a foot switch 14 so that the operation of an ozone water production module 12 can be controlled. When the foot switch 14 is provided, it becomes possible to conduct the start and stop, or the like of production and supply of ozone water from an ejection nozzle even during surgery with a handpiece 8 in hand by manipulating the foot switch 14 while conducting the surgery. The handpiece 8 may be an irrigator that is provided with an ejection nozzle ejecting ozone water toward the desired site in oral cavity. Moreover, the handpiece 8 may be a controller for an external water feeding type implant by which ozone water is supplied through a tube, and the ejection nozzle may be made so that when a tool is fit to the controller for an external water feeding type implant to conduct drilling of a bone, ozone water is ejected toward a tip of the tool. In this case, it is possible to conduct disinfection and sterilization at the site of surgery while utilizing ozone water for cooling the heat generated at the site of surgery at the time of drilling of the bone with the tool.

In Figure 2, the ozone water production module 12 is a device that produces ozone water from water such as tap water or purified water with electric power supplied from a power supply 9. The ozone water cleaning apparatus 3 includes a water reservoir tank 10, a pump 11, and the ozone water production module 12, water that is reserved in the water reservoir tank 10 is fed to the ozone water production module 12 by the pump 11, and the ozone water production module 12 produces ozone water from the fed water.

The water reservoir tank 10 is for reserving water and is provided with a water inlet that can be opened and closed with a cap 13, and tap water or the like can be replenished to the water reservoir tank 10 through the water inlet. Moreover, the pump 11 is for feeding water that is reserved in the water reservoir tank 10 to the ozone water production module 12, and pumps of any type can be used as long as water can be fed. However, as will be described later, it is preferable that the pump 11 is made so that the water feeding capacity thereof can be changed for the purpose of making it easy to adjust the dissolved ozone concentration of ozone water.

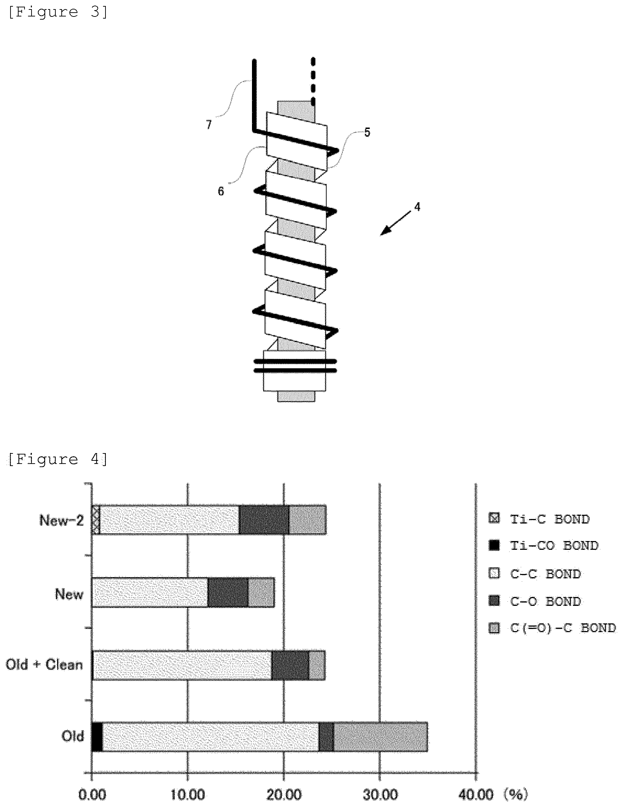

Figure 3 illustrates an example of the electrolytic ozone water production apparatus 1 that is used in the present invention, and an electrolysis unit 4 is housed in the electrolytic ozone water production apparatus 1. The electrolysis unit 4 is constituted by winding a band of a diaphragm 6 made of an ion exchange membrane around an anode 5 that is a metal bar in which a conductive diamond catalyst is supported and winding a cathode 7 made of a metal wire around the diaphragm 6.

### [Electrolysis Conditions]

As electrolysis conditions, it is preferable from the standpoint of stability and activity of generated substances that the temperature is from 5°C to 40°C, and it is preferable that the direct current for applying to an electrolysis cell is 0.01 to 1 A/cm² in terms of current density to a plane of projection for an anode. The flow rate of water in this case is adjusted so that an intended ozone concentration is obtained. It is possible to complete the hydrophilization treatment in a shorter time as the concentration of ozone water is higher, and the concentration is from 1 ppm to 25 ppm. For example, the flow rate of water may be changed from 1 L/min to 50 mL/min in order to adjust the ozone concentration from 1 ppm to 25 ppm for a cell to which a current of 1 A can be applied.

Removal of carbide with ozone water can be completed in a short time when the concentration of ozone water is high, and a treatment time of 5 to 10 minutes is most preferable. It is preferable to maintain the temperature of the implant material at around room temperature (about 25°C) when used in oral cavity as it is because a burden to patients is too large when the implant material is too cold. The carbon content in the carbide adsorbed on the surface of the implant material is made less than 25 atomic%, preferably less than 20 atomic%. Here, the carbon content (atomic%) in the carbide adsorbed on the surface of the implant material is determined by conducting surface analysis to measure the concentration of elements in a volume of φ 100 µm × 3 nm on the sample surface by XPS (product name "XPS-PHI5000 Versa Probe" manufactured by ULVAC-PHI, INCORPORATED).

### [Ozonized Water]

Ozone water is a product of electrolysis in which an ozone gas obtained through electrolysis of pure water, tap water, or the like using an electrolysis cell is mainly dissolved, however, in the present invention, the ozone water means ozone gas-containing water that contains, in addition to the ozone gas, an oxygen radical such as an OH radical or a super oxide anion; hydrogen peroxide; or another oxidizing substance. Regarding the oxidative effect of ozone water, oxidation is mainly caused by the ozone gas itself at a low pH (acidic pH), and, at a high pH (alkaline pH), the ozone gas decomposes and oxidation is mainly caused by an OH radical that is generated at the time of decomposition of the ozone gas, and therefore the oxidative effect of ozone water becomes further strong as compared with other oxidizing substances the oxidizing equivalences of which are the same as ozone water.

### [Method and Apparatus for Producing Ozone Water]

Ozone water, in general, has the following characteristics.
(1) The disinfection effect by ozone (OH radical) is an oxidative destruction of cell walls and is indiscriminate, and therefore it can be said that a resistant bacterium does not exist.
(2) Oxygen is produced by decomposition and no residue is left.

Ozone water has generally been produced using a discharge type ozone gas generator so far, and ozone water having an ozone concentration of several ppm can easily be produced and has been utilized in the fields such as water-cleaning treatment and food cleaning. However, the discharge type ozone gas generator has been inappropriate as a handy generator for ozone water apparatus producing high-concentration ozone water, the generator having an excellent instantaneous responsiveness because of the following reasons.
(1) Two steps, an ozone gas-generating step of temporarily generating ozone as a gas and a dissolving step of dissolving the ozone gas after the ozone gas generation, are necessary.
(2) Produced ozone water has a lower concentration as compared with ozone water produced by an electrolytic method as will be described later, and therefore ozone water needs to be produced by injecting ozone into water under high pressure to dissolve the ozone.
(3) It is hard to make the ozone gas generator small because a power supply for ozone gas generation requires high voltage/high frequency.
(4) A ozone water production apparatus by means of discharge takes some time (a few minutes of standby time) until the ozone gas generation capacity becomes stable, and it is hard to prepare ozone water having a constant concentration instantaneously.

By an anode reaction in the electrolysis cell, oxygen generation progresses as shown by the following equation (1) in the case of water only, ozone is produced as shown by the following equation (2) depending on the catalyst or the electrolysis conditions, and ozone water dissolving the ozone can be synthesized.

2H₂O = O₂ + 4H⁺ + 4e⁻ (1)

3H₂O = O₃ + 6H⁺ + 6e⁻ (2)

The electrolytic method is inferior to the discharge method in terms of power consumption, however the electrolytic method has a characteristic that a high-concentration ozone gas and ozone water can easily be obtained and therefore has been used for general purposes in special fields such as cleaning of electronic parts. The electrolytic method uses a low-voltage DC power supply in principle and therefore is excellent in instantaneous responsiveness and safety, and utilization of the electrolytic method as a small-sized ozone gas or ozone water generator is expected. Moreover, any of a battery-driven type, a power generator-driven type, and an AC/DC converter-driven type can be selected in accordance with the intended use.

### [Anode/Substrate]

It is essential to select an appropriate catalyst and electrolyte in order to efficiently generate the ozone gas. As an electrode material, noble metals such as platinum; α-lead dioxide; β-lead dioxide; glassy carbons in which fluorocarbons are supported; and conductive diamond are known. It is preferable that a material which is usable as an electrode substrate has long life and corrosion resistance so that the contamination on the treated surface does not occur, and Si (monocrystal, polycrystal), Nb, Ta, Zr, Ti, Mo, W, graphite, and so on are usable and can be selected depending on the intended use.

### [Cathode/Substrate]

The cathode reaction is mainly hydrogen generation, an electrode catalyst that is not embrittled by hydrogen is preferable, and platinum group metals, Ni, stainless steel titanium, zirconium, gold, silver, carbon, diamond, and so on are preferable. As a cathode substrate, use of stainless steel, zirconium, carbon, nickel, titanium, or the like is desirable.

### [Diaphragm Material]

As a diaphragm, ion exchange membranes and neutral membranes are usable, and the ion exchange membranes are usually used. The diaphragm has a function of allowing electrolysis to quickly progress even when the conductivity of a solution is low in addition to a function of preventing the substances produced at the anode or the cathode from being consumed at the opposite electrode, and therefore the diaphragm is preferably usable when pure water or the like that has a poor conductivity is utilized as a raw material. When the ion exchange membrane is used, any of fluororesin-based and hydrocarbon resin-based ion exchange membranes may be used, however the former is preferable in terms of resistance to corrosion by ozone or peroxides. It is preferable that the membrane thickness is from 0.1 to 1 mm.

### [Membrane-Electrode Assembly]

As an electrode, it is preferable to use conductive diamond, and an electrolysis cell obtained by disposing a band-shaped diaphragm member on a bar-shaped conductive diamond electrode and further disposing a wire-shaped counter electrode thereon is usable. Moreover, in the membrane-electrode assembly, a diaphragm such as an ion exchange membrane is provided around a bar-shaped or cylindrical electrode, usually anode (hereinafter, also referred to as bar-shaped anode); a wire-shaped counter electrode or a porous counter electrode, usually a wire-shaped cathode or a porous cathode, is disposed around the membrane; the membrane and the anode are fixed using the cathode so that at least a part of the membrane and a part of the anode make contact each other and at least a part of the membrane and a part of the cathode make contact with each other; and thereby an anode chamber having a gas/liquid channel between the membrane and the anode, further preferably between a plurality of anodes can be formed, and the electrode-membrane assembly has a suitable cell structure.

The length and diameter of the bar-shaped anode in the membrane-electrode assembly are determined in accordance with the required amount of ozone. Usually, it is preferable that the length is from 10 mm to 300 mm and the diameter is from 0.5 mm to 10 mm. The diameter of the diaphragm in the assembly is set so as to be larger than the diameter of the bar-shaped anode (representatively, a column is supposed) that is housed in the assembly by an amount of from 0.1 mm to 5 mm. It is preferable that the opening ratio of the porous cathode is from 20% to 80% and the thickness is from 0.1 mm to 2 mm.

When the wire-shaped cathode (feeder line) is used, it is preferable that the diameter is in a range of 0.1 mm to 2 mm. When the wire-shaped cathode is thinner than 0.1 mm, voltage loss due to electric resistance cannot be ignored and the wire-shaped cathode becomes liable to be cut during winding work because of a shortage of physical strength. On the other hand, when the wire-shaped cathode is too thick, the substance movement of raw materials or products of electrolysis from the anode chamber is suppressed, and an increase in voltage or a decrease in current efficiency is brought about and it becomes hard to conduct wiring work. When the wire-shaped cathode or feeder line is spirally wound on the outside of the anode and the membrane, it is suitable that the interval of cathode line is from about 0.1 mm to about 10 mm. As for the membrane, it is preferable to utilize a membrane the mechanical strength of which is strengthened with reinforcing fiber.

When the cylindrical diaphragm is necessary, it is suitable that the diaphragm is molded in advance. The molding can easily be conducted by a publicly known tube-molding process using a precursor resin having a thermoplastic property. It is preferable that the diaphragm is the one in which the reinforcing fiber is used. A cylindrical diaphragm may be directly formed from a sheet-like diaphragm and thereafter adhered. A fluororesin-based ion exchange membrane can be fixed with the one edge being placed on top of the other by thermal fusion or with an adhesive agent. It is adequate regarding the thermal fusion that the treatment temperature is in a range of from 200°C to 350°C, the bearing pressure is in a range of from 2 kg/cm² to 20 kg/cm², and the time is in a range of from 1 second to 1 minute. It is suitable that the adhesion is conducted with a thin band of the fluroresin-based membrane which does not contain reinforcing fiber interposed in between for the purpose of enhancing connection strength and achieving more complete connection. It is suitable that unevenness is provided on the surface of the membrane because gas/liquid permeability can be enhanced.

The above-described dimensions are determined and designed from the standpoint of necessity that at least a part of each electrode and a part of the membrane make a close contact with each other spirally to thereby make it possible for electrolysis to progress smoothly even in the case of raw material water having a small conductivity and that the anode chamber made up of the anode and the membrane has a volume in which quick flow of supplied raw material water and a generated gas component is possible.

### [Electrolysis Cell]

At least one opening part of the anode chamber that is made up of the anode and the diaphragm in the membrane-electrode assembly is fixed to a tube that is connected to a path of the raw material water. The tube has a diameter about the same as that of the cylindrical diaphragm, the diaphragm and the tube is fixed with an adhesive agent, and a feeder terminal of the bar-shaped anode is connected to the anode in the tube.

Since raw material water that is not sufficiently electrolyzed is contained in water flowing out from the electrolysis cell at first, it is more preferable that the amount of water which is present in the electrolysis cell and the volume of piping parts other than the electrolysis cell are smaller. Dissolving of gasified ozone in the raw material water progresses, and the production efficiency of the ozone water is increased. Therefore, it is suitable to set the length of the bar-shaped anode as the optimal length to a range of from 0.1 seconds to 10 seconds in terms of contact time.

### [Raw Material Water and Produced Electrolyzed Water]

Purified water, pure water, tap water, well-water, and so on can be used as raw material water. In a treatment target that contains a large amount of metal ion such as Ca or Mg, as is the case in the latter two examples of the raw material water, there is a risk that reaction is inhibited due to precipitation of hydroxides or carbonates on the surface of the cathode. Moreover, oxides such as silica are precipitated on the surface of the anode. When a reverse current is applied at an appropriate time interval (1 minute to 1 hour) as a measure for preventing the precipitation, acidification occurs at the cathode and alkalization occurs at the anode, and therefore detachment reaction of precipitates easily progresses by the flow of the generated gas and supplied water.

In the present invention, it is preferable that the raw material water is pure water so that the contamination does not occur to the implant material. When pure water is utilized, electrolysis performance becomes stable and the apparatus can be utilized over a long period of time.

### [Materials of Entire Apparatus]

A material that is not damaged by raw material water is selected as a material of a tank as a container that reserves raw material water and a material of piping. A PE resin may be used if there is no particular problem. It is preferable to supply raw material water through a pump directly from a pure water production apparatus.

### Examples

Hereinafter, Examples of the present invention will be shown.

### <Example 1>

A band of an ion exchange membrane (Nafion 350 manufactured, thickness 0.35 mm, and diameter 3 mm) (Nafion is a trade mark of DuPont) was spirally wound around a niobium bar (diameter 2 mm) on which a conductive diamond catalyst (doped boron concentration 2500 ppm) was formed as an anode, and a platinum wire was wound on the diaphragm, thereby making an anode-membrane-cathode assembly. Pure water was allowed to flow by 40 mL per minute from the lower part of the electrolysis cell to which the membrane-electrode assembly was fit. When a current of 0.5 A was applied, since the diaphragm was spiral, oxygen and ozone which were generated from the anode and a hydrogen gas which was generated from the cathode were mixed to produce electrolyzed water in which oxygen, ozone, and the hydrogen gas were dissolved, and, at the time of the electrolyzed water production, a cell voltage was 10 V and the concentration of ozone water was 4 ppm.

Acid etching treatment was applied to a surface of a titanium disk having a diameter of 8 mm and a thickness of 1 mm, then the disk was stored in a petri dish and left standing for nine months at room temperature. The acid etching treatment was conducted by immersing the titanium disk in a 48% sulfuric acid solution at a temperature of 60°C for 10 minutes. Immediately after the acid etching treatment, ultrasonic cleaning was conducted twice in a sufficient amount of pure water. Surface analysis by XPS was conducted to the sample that had been left standing for nine months. The XPS-PHI5000 Versa Probe manufactured by ULVAC-PHI, INCORPORATED was used for XPS. Surface analysis was conducted by XPS to compare the concentrations of elements in a volume of φ 100 µm × 3 nm on the sample surface. The surface was cleaned with the above-described ozone water having an ozone concentration of 4 ppm in flowing water for 10 minutes, thereafter the sample was sufficiently dried, and reanalysis was conducted. The results are shown in Table 1 and Figure 4.

**[Table 1]**

| | Ti-C bond (281.5 eV) | Ti-CO bond (283 eV) | C-C bond (285 eV) | C-O bond (286.6 eV) | C(=O)-O bond (288.6 eV) | Total [%] |
|---|---|---|---|---|---|---|
| Old | 0.00 | 1.20 | 22.40 | 1.67 | 9.73 | 35.00 |
| Old + Clean | 0.00 | 0.11 | 18.64 | 3.74 | 1.80 | 24.29 |
| New | 0.00 | 0.00 | 12.17 | 4.16 | 2.64 | 18.97 |
| New-2 | 0.58 | 0.00 | 14.78 | 5.22 | 3.82 | 24.40 |

New and New-2 denote the samples 4 days after the treatment of the titanium surface, Old denotes a sample after being left standing for nine months, and Old + Clean denotes a sample obtained by cleaning the Old sample with ozone, however, as a result of the treatment, the carbon content on the surface returns to a state about the same as that of the sample after 4 days. The amounts of a C-C bond and a C(=O)-O bond were decreased, however the amount of a C-O bond was increased. The amount of carbon compounds as a whole was decreased by about 30%, and the details were that the amounts of the C-C bond and the C(=O)-O bond were decreased and the amount of the C-O bond was increased. It is considered that the reason is because although the amounts of the C-C bond and the C(=O)-O bond were decreased due to active species in ozone water, the amount of the C-O bond was increased due to binding between the active species and carbon compounds. However, the carbon compounds as a whole was decreased by about 30% to obtain a surface having a carbon content of less than 25 atomic%, and it was suggested that the cleaning effect by ozone water be exhibited. Differences to the bond strength were quantitatively confirmed by conducting effect checking tests using rats.

### <Example 2>

A niobium bar (diameter 2 mm) on which a conductive diamond catalyst (doped boron concentration 2500 ppm) was formed as an anode was put into an ion exchange membrane (Nafion 350 manufactured by DuPont, thickness 0.35 mm, and diameter 3 mm), and a commercially available platinum wire (diameter 0.4 mm) was spirally wound as a cathode on the membrane, thereby making an anode-membrane-cathode assembly. The spiral interval was 4 mm. A tube (diameter 4 mm) was adhered to both of the upper part and the lower part of the assembly, feeder wires from a DC power supply were connected to respective electrodes, thereby making an electrolysis cell, and pure water was allowed to flow by 40 mL per minute from the lower part of the cathode chamber. When a current of 1 A was applied, a cell voltage at the time of applying the current was 19 V and the concentration of ozone water at the time of applying the current was 21 ppm.

When the same experiment as in Example 1 was conducted, the amount of carbon compounds on the titanium surface was decreased by 50% to obtain a surface having a carbon content of less than 20 atomic%, and it was suggested that the cleaning effect by ozone water be exhibited. Differences to the bond strength were quantitatively confirmed by conducting effect checking tests using rats.

### Industrial Applicability

The present invention can be applied to a method for treating an implant material having excellent biocompatibility, and particularly to a method for treating a dental implant material.

### Reference Signs List

- 1:: Electrolytic ozone water production apparatus
- 2:: Implant hydrophilization treatment container
- 3:: Ozone water cleaning apparatus
- 4:: Electrolysis unit
- 5:: Anode
- 6:: Diaphragm
- 7:: Cathode
- 8:: Handpiece
- 9:: Power supply
- 10:: Water reservoir tank
- 11:: Pump
- 12:: Ozone water production module
- 13:: Cap
- 14:: Foot switch

## Claims

1. A method for treating an implant material having excellent biocompatibility, the method comprising:
immersing a surface-roughened implant material comprising titanium or a titanium alloy in electrolyzed ozone water having an ozone concentration of from 1 ppm to 25 ppm with a contact time from 1 minute to 60 minutes; and
maintaining the electrolyzed ozone water at normal temperature,
thereby treating the implant material by the electrolyzed ozone water and preventing contamination due to adsorption of carbide on the surface of the implant material and imparting hydrophilicity to the surface of the implant material, wherein a carbon content in the carbide adsorbed on the surface of the implant material after treatment is made less than 25 atomic%.

2. A non-surgical method according to claim 1 further comprising:
supplying electrolyzed ozone water having an ozone concentration of from 0.2 ppm to 5 ppm to the surface of the implant material just before the implant surgery, thereby preventing contamination due to adsorption of carbide on the surface of the implant material and imparting hydrophilicity to the surface of the implant material.

3. The method for treating an implant material having excellent biocompatibility according to Claim 1 or 2, wherein a carbon content in the carbide adsorbed on the surface of the implant material after treatment is made less than 20 atomic%.

4. The non-surgical method for treating an implant material having excellent biocompatibility according to Claim 2 or 3, wherein ozone water having an ozone concentration of from 0.3 ppm to 5 ppm is used as the electrolyzed ozone water supplied to the surface of the implant material just before the implant surgery.

5. The method for treating an implant material having excellent biocompatibility according to any one of Claims 1 to 4, wherein the implant material is an implant material which has lost biocompatibility due to contact with air.

6. The method for treating an implant material having excellent biocompatibility according to any one of Claims 1 to 5, wherein the implant material is a dental implant material.

## Patentansprüche

1. Verfahren zur Behandlung eines Implantat-Materials, das eine ausgezeichnete Biokompatibilität aufweist, wobei das Verfahren Folgendes umfasst:
Eintauchen eines Implantat-Materials mit aufgerauter Oberfläche, das Titan oder eine Titanlegierung umfasst, in elektrolysiertes, ozonisiertes Wasser, das eine Ozonkonzentration von 1 ppm bis 25 ppm aufweist, mit einer Kontaktzeit von 1 Minute bis 60 Minuten; und
Halten des elektrolysierten, ozonisierten Wassers bei Normaltemperatur,
wodurch das Implantat-Material durch das elektrolysierte, ozonisierte Wasser behandelt wird und eine Kontamination aufgrund der Adsorption von Carbid auf der Oberfläche des Implantat-Materials verhindert wird und der Oberfläche des Implantat-Materials Hydrophilie verliehen wird, wobei der Kohlenstoffgehalt in dem an der Oberfläche des Implantat-Materials adsorbierten Carbid nach der Behandlung weniger als 25 Atomprozent beträgt.

2. Nicht-operatives Verfahren nach Anspruch 1, ferner umfassend:
Zuführen von elektrolysiertem, ozonisiertem Wasser, das eine Ozonkonzentration von 0,2 ppm bis 5 ppm aufweist, zu der Oberfläche des Implantat-Materials kurz vor der Implantat-Operation, damit eine Kontamination aufgrund der Adsorption von Carbid auf der Oberfläche des Implantat-Materials verhindert wird und der Oberfläche des Implantat-Materials Hydrophilie verliehen wird.

3. Verfahren zur Behandlung eines Implantat-Materials, das eine ausgezeichnete Biokompatibilität aufweist, nach Anspruch 1 oder 2, wobei der Kohlenstoffgehalt in dem an der Oberfläche des Implantat-Materials adsorbierten Carbid nach der Behandlung weniger als 20 Atomprozent beträgt.

4. Nicht-operatives Verfahren zur Behandlung eines Implantat-Materials, das eine ausgezeichnete Biokompatibilität aufweist, nach Anspruch 2 oder 3, wobei ozonisiertes Wasser, das eine Ozonkonzentration von 0,3 ppm bis 5 ppm aufweist, als elektrolysiertes, ozonisiertes Wasser verwendet wird, das der Oberfläche des Implantat-Materials kurz vor der Implantat-Operation zugeführt wird.

5. Verfahren zur Behandlung eines Implantat-Materials, das eine ausgezeichnete Biokompatibilität aufweist, nach einem der Ansprüche 1 bis 4, wobei das Implantat-Material ein Implantat-Material ist, das die Biokompatibilität aufgrund des Kontakts mit Luft eingebüßt hat.

6. Verfahren zur Behandlung eines Implantat-Materials, das eine ausgezeichnete Biokompatibilität aufweist, nach einem der Ansprüche 1 bis 5, wobei das Implantat-Material ein zahnmedizinisches Implantat-Material ist.

## Revendications

1. Procédé pour traiter un matériau d'implant présentant une excellente biocompatibilité, le procédé comprenant :
l'immersion d'un matériau d'implant dont la surface a été rendue rugueuse, comprenant du titane ou un alliage de titane, dans de l'eau ozonisée électrolysée possédant une concentration d'ozone de 1 ppm à 25 ppm avec un temps de contact de 1 minute à 60 minutes ; et
le maintien de l'eau ozonisée électrolysée à la température normale,
en conséquence de quoi le matériau d'implant est traité par l'eau ozonisée électrolysée et une contamination du fait de l'adsorption de carbure sur la surface du matériau d'implant est empêchée et un caractère hydrophile est conféré à la surface du matériau d'implant,
dans lequel la teneur en carbone du carbure adsorbé sur la surface du matériau d'implant après le traitement est rendue inférieure à 25 % atomiques.

2. Procédé non chirurgical selon la revendication 1, comprenant en outre :
la fourniture d'eau ozonisée électrolysée, ayant une concentration d'ozone de 0,2 ppm à 5 ppm, à la surface du matériau d'implant juste avant une chirurgie d'implantation, en conséquence de quoi une contamination du fait de l'adsorption de carbure sur la surface du matériau d'implant est empêchée, et un caractère hydrophile est conféré à la surface du matériau d'implant.

3. Procédé pour traiter un matériau d'implant présentant une excellente biocompatibilité selon la revendication 1 ou 2, dans lequel la teneur en carbone du carbure adsorbé sur la surface du matériau d'implant après le traitement est rendue inférieure à 20 % atomiques.

4. Procédé non chirurgical pour traiter un matériau d'implant présentant une excellente biocompatibilité selon la revendication 2 ou 3, dans lequel de l'eau ozonisée ayant une concentration d'ozone de 0,3 ppm à 5 ppm est utilisée en tant qu'eau ozonisée électrolysée fournie à la surface du matériau d'implant juste avant la chirurgie d'implantation.

5. Procédé pour traiter un matériau d'implant présentant une excellente biocompatibilité selon l'une quelconque des revendications 1 à 4, dans lequel le matériau d'implant est un matériau d'implant qui a perdu sa biocompatibilité du fait d'un contact avec l'air.

6. Procédé pour traiter un matériau d'implant présentant une excellente biocompatibilité selon l'une quelconque des revendications 1 à 5, dans lequel le matériau d'implant est un matériau d'implant dentaire.
